# EUROPEAN PATENT SPECIFICATION

(11) **EP 1 991 561 B1**
(45) Date of publication and mention of the grant of the patent: **17.06.2015**
(21) Application number: 07722808.8
(22) Date of filing: 13.02.2007
(51) Int. Cl.: C07K 7/08, C07K 7/23, A61K 38/09, A61K 38/10, G01N 33/68, A61P 35/00

(54) **BRANCHED MULTIMERIC PEPTIDES FOR TUMOR DIAGNOSIS AND THERAPY**
VERZWEIGTE MULTIMERE PEPTIDE ZUR TUMORDIAGNOSE UND THERAPIE
PEPTIDES MULTIMÈRES BRANCHÉS POUR UN DIAGNOSTIC DE TUMEUR ET UNE THÉRAPIE

(30) Priority: 14.02.2006 US 773693 P
(43) Date of publication of application: 19.11.2008
(73) Proprietor: Universita' Degli Studi di Siena, I-53100 Siena (IT)
(72) Inventor: BRACCI, Luisa, I-53100 Siena (IT); FALCIANI, Chiara, I-53100 Siena (IT); PINI, Alessandro, I-53100 Siena (IT); FABBRINI, Monica, I-53100 Siena (IT)
(74) Representative: Capasso, Olga
(86) International application number: PCT/EP2007/001224
(87) International publication number: WO 2007/093373

(56) References cited:
- WO-A-96/40755
- US-A- 5 885 966
- US-A1- 2004 202 673
- BRACCI LUISA ET AL: "Synthetic peptides in the form of dendrimers become resistant to protease activity." THE JOURNAL OF BIOLOGICAL CHEMISTRY 21 NOV 2003, vol. 278, no. 47, 21 November 2003 (2003-11-21), pages 46590-46595, XP002461778 ISSN: 0021-9258
- HULTSCH CHRISTINA ET AL: "Synthesis and evaluation of novel multimeric neurotensin(8-13) analogs." BIOORGANIC & MEDICINAL CHEMISTRY 1 SEP 2006, vol. 14, no. 17, 1 September 2006 (2006-09-01), pages 5913-5920, XP002461779 ISSN: 0968-0896

## Description

The instant invention refers to in vivo stable branched peptides, in particular derived from the sequence of Neurotensin (NT), conjugated to functional units for specific targeting of cancer cells, either for diagnosis or therapy of tumors expressing NT.

### Background art

One of the major problems in classic chemotherapy is the non-specific toxicity of most anticancer agents against normal cells. The specific targeting of tumors has been the main challenge in the research on cancer therapy and diagnosis.

Presently, innovative tumor-specific therapies follow the strategy of targeting tumor associated proteins, specifically expressed or over expressed on tumor cells.

The observation that receptors for different endogenous regulatory peptides are expressed in a number of primary human cancers, opened new perspectives on the use of synthetic peptides for tumor-selective targeting¹⁻³.

Neurotensin (NT) is a 13 amino acid peptide (QLYENKPRRPYIL) originally isolated from calf hypothalamus⁶. It has the dual function of neurotransmitter or neuromodulator in the nervous system and local hormone in the periphery. NT receptors are overexpressed in severe malignancies like small cell lung cancer, colon, pancreatic and prostate carcinomas. NT stabilized analogues have been proposed for tumor therapy since several years ago⁴⁻¹⁰ and NT is still considered the best possible candidate for peptide-based therapy of exocrine pancreatic carcinomas¹¹ in consideration of the high incidence and density of NT receptors in these tumors. Over 75% of all ductal pancreatic carcinomas over-express NT receptors, whereas normal pancreas tissue, pancreatitis and endocrine pancreas do not¹².

The presence of specific receptors on membranes of various tumor cells makes analogues of NT excellent choices as carrier molecules. Tumor receptor-targeting is fundamental in approaching the problem of non-specific toxicity of cancer chemotherapies and it is a precious tool for tumor localization by radioisotopes. Nonetheless, the use of peptides *in vivo* has largely been limited by their short half-life.

The inventors previously demonstrated that synthesis of peptides in branched multimeric form results in molecules that can retain peptide biological activity and are very resistant to proteolytic activity of biological fluids, thus having a markedly higher half-life in respect of correspondent monomeric peptides¹⁴.

The instant invention refers to in vivo stable multimeric peptides derived from NT conjugated to functional units for specific targeting of cancer cells, either for diagnosis or therapy of tumors expressing NT.

### Description of invention

It is an object of the present invention, a multimeric peptide having the general formula A or B. 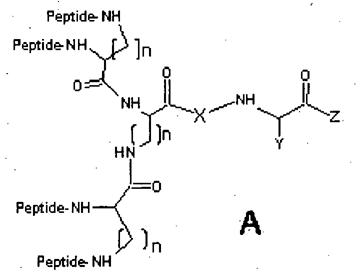 or 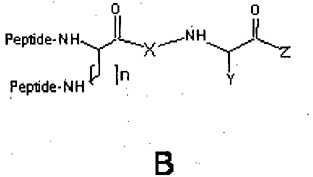 wherein
n = 1 to 5,
X = 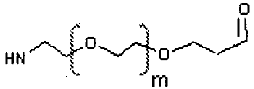 wherein m = 1 to 27,
or 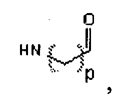 wherein p= 1 to 5, 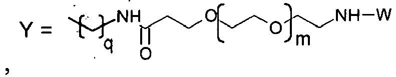 wherein m = 1-27 and q= 1 to 5,
or 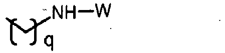 wherein q= 1 to 5,
or 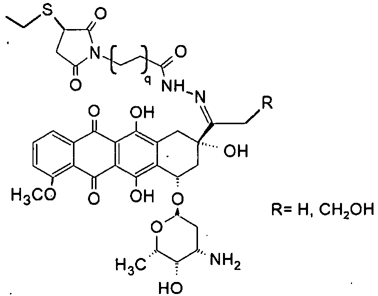 wherein q= 1 to 5
W = 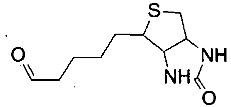 or 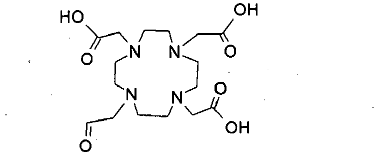 or 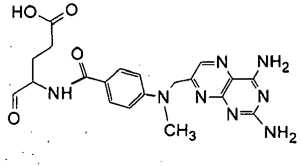 or 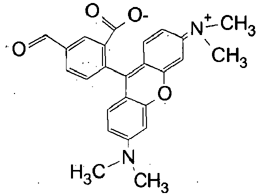 or 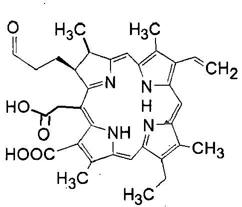 and Z = NH₂ or OH,
wherein each peptide has essentially a Neurotensin (NT) derived sequence, for use as an anti-tumoral agent.

Preferably the multimeric peptide for use as an anti-tumoral agent, wherein each peptide having a Neurotensin (NT) derived sequence has essentially the following amino acid sequence: QLYENKPRRPYIL or RRPYIL. In a preferred embodiment the multimeric peptide for use as an anti-tumoral agent is conjugated with a functional unit.

It is an object of the present invention a pharmaceutical composition comprising the multimeric peptide, or a pharmaceutically acceptable and efficient salt thereof and diluents, and/or solvents and/or carriers and/or eccipients.

It is an object of the present invention, the use of the multimeric peptide for tumor diagnostics, preferably for tumor expressing neurotensin.

It is an object of the present invention a method to in vitro diagnose a tumor in a sample comprising the steps of:
a) incubating the sample with the multimeric peptide in proper conditions to allow the specific binding of the peptide to the sample, and
b) detecting the bound peptide.

Preferably the tumor expresses neurotensin receptors, more preferably the tumor is a small cell lung, a colon, a pancreatic, a prostate, an endometrial or an ovarian carcinoma, or a breast cancer.

The invention will be now described by non limiting examples referring to the following figures:

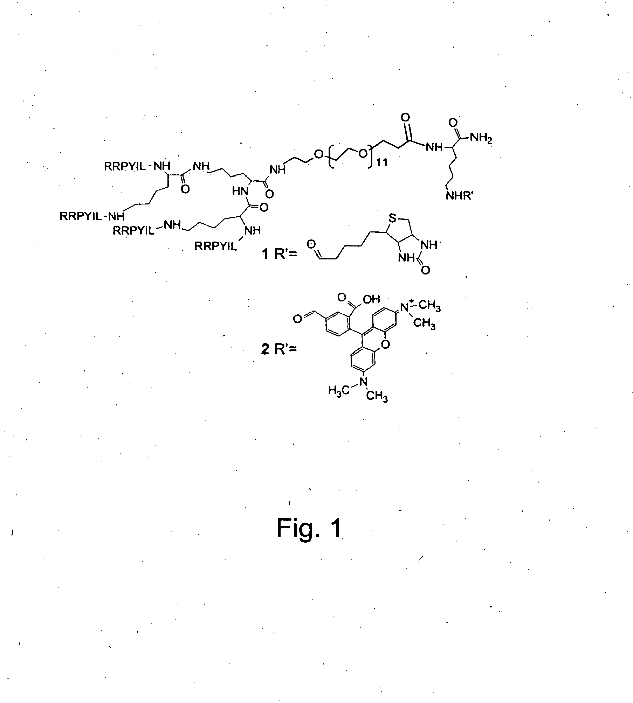

**FIGURE 1****.** Structure of **1:** tetrameric NT(8-13)4-PEG-Biotin (NT4(8-13)-Bio) and **2:** tetrameric NT(8-13)4-PEG-Tetramethyl rhodamine (NT4(8-13)-TMR).

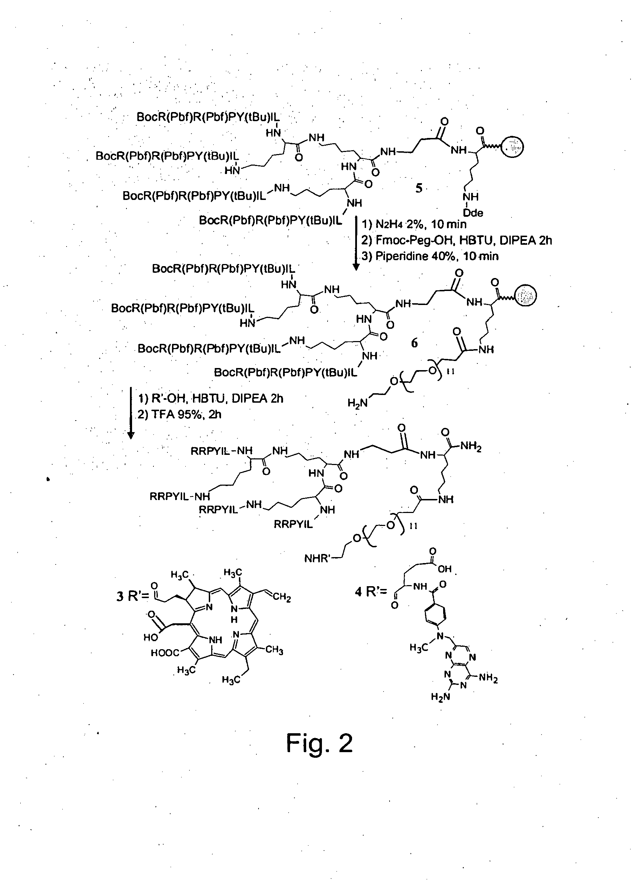

**FIGURE 2****.** Synthesis of **3:** tetrabranched NT(8-13)4-PEG-Chlorine e6 (NT4(8-13)-Che6) and 4: tetrabranched NT(8-13)-PEG-Methotrexate (NT4(8-13)-MTX).

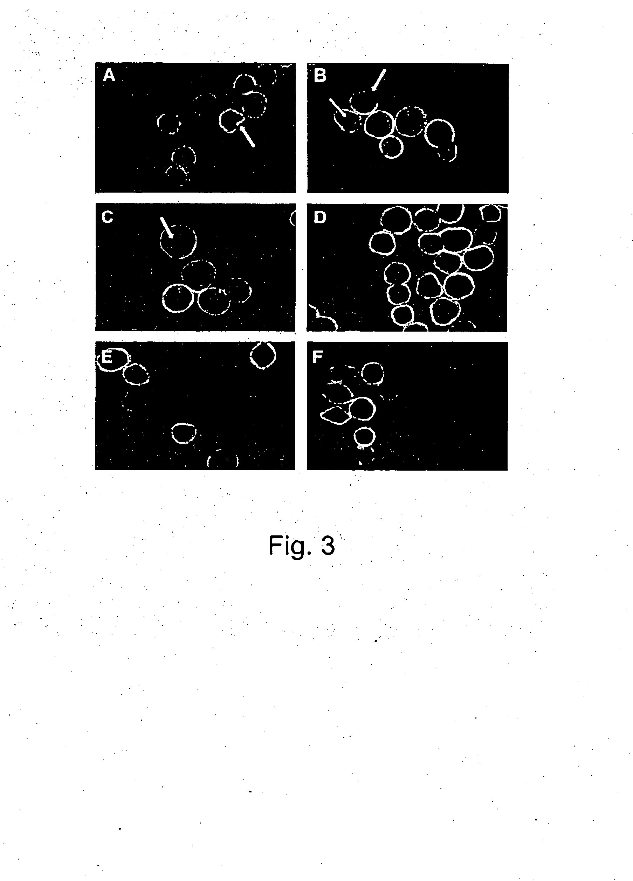

**FIGURE 3****.** NT4(8-13) peptide binding and cell internalization. Confocal microscopy images of HT29 cells incubated with NT4(8-13)-TMR (5 µg/ml in PBS-BSA1%) for 1 h at 4°C to allow peptide binding, washed and then incubated at 37°C with previously warmed medium for 0 h (panel A, NT4(8-13)-TMR is on the surface of the membrane, see arrow), 2 h (panel B, NT4(8-13)-TMR is on the surface of the membrane and inside the cell, see arrows), 4h (panel C, NT4(8-13)-TMR is only inside the cell, see arrow) and 18 hours (panel D, no NT4(8-13)-TMR signal). After fixing with 4% formalin, the plasma membrane was stained in green with concanavalin A-biotin and avidin -FITC. Similar experiments were carried out incubating HT29 cells with scramble-NT4(8-13)-TMR (panel E, no rhodamine signal) and HaCat cells with NT4(8-13)- TMR (panel F, no rhodamine signal) for 1h at 4°C.

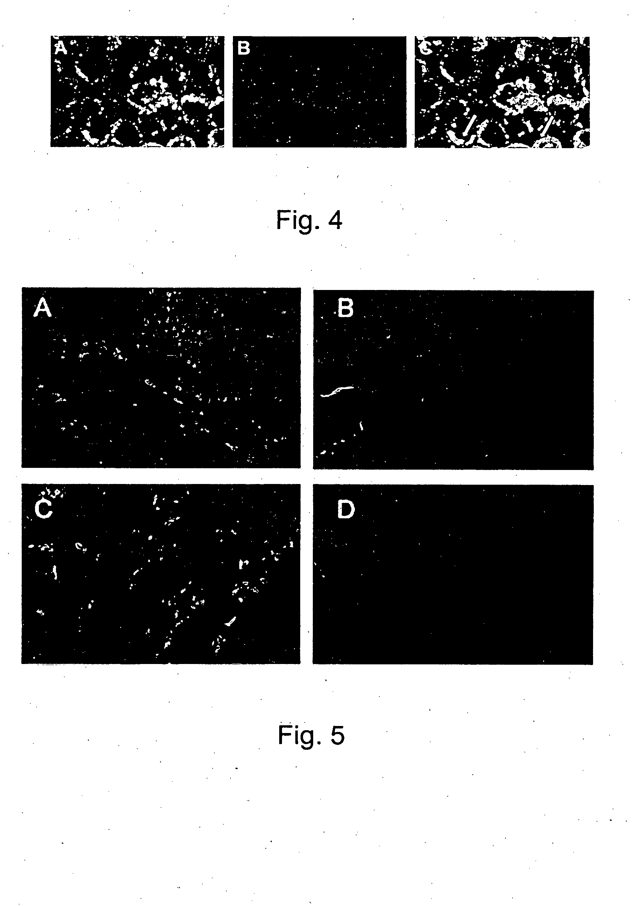

**FIGURE 4****.** Lysosome localization of NT4(8-13)-TMR. HT29 cells were incubated with NT4(8-13)-TMR under internalizing conditions (1 h incubation at 4°C with NT4(8-13)-TMR followed by 4 hours incubation in DMEM at 37°C) and then treated with lysotracker green to stain lysosomes. Panel A shows lysotracker green, panel B shows the red signal of the peptide and panel C shows their colocalization that gives a yellow signal (see arrows).
**FIGURE 5****.** NT4(8-13) binding to colon and pancreas tumoral biopsies. Confocal microscopy images of (A) human sections of colon carcinoma, (B) healthy colon, (C) pancreatic carcinoma and (D) healthy pancreas. After fixing with 4% paraformaldehyde and saturation with FCS, the sections were incubated with NT4(8-13)-biotin (0.5 µg/ml in PBS-BSA3%) for 30' at 37°C, washed twice with PBS and incubated with avidin-FITC (0.5 µg/ml in PBS-BSA3%) for 30'at room temperature.

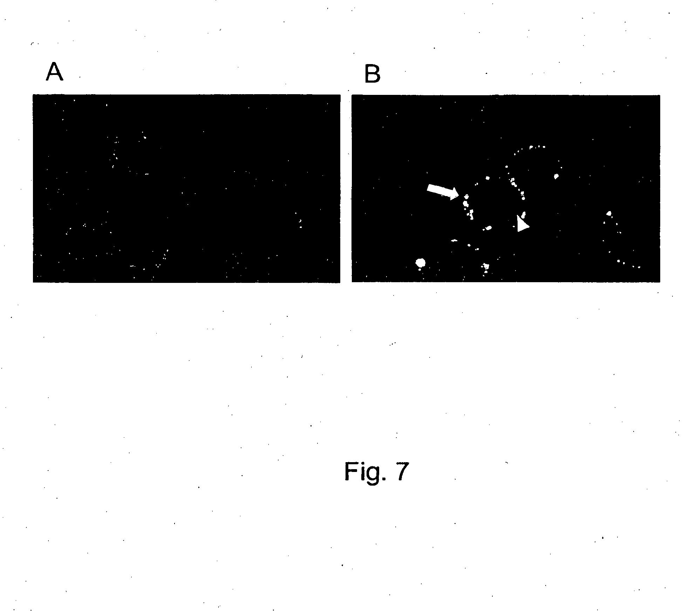

**FIGURE 7****.** NTR regeneration. (A) HT29 cells first incubated with NT4(8-13)-TMR (100 µg/ml) for 1 h at 4°C and then, after washing, with NT4(8-13)-Bio followed by avidin-FITC, only red staining of NT4(8-13)-TMR is visible. (B) HT29 cells incubated with NT4(8-13)-TMR as in (A), washed, kept for 18 h at 37°C in culture medium and treated with NT4(8-13)-Bio and avidin-FITC. The arrow head indicates the red localization of NT4(8-13)-TMR, the complete arrow indicates the green signal of NT4(8-13)-Bio-avidin FITC.

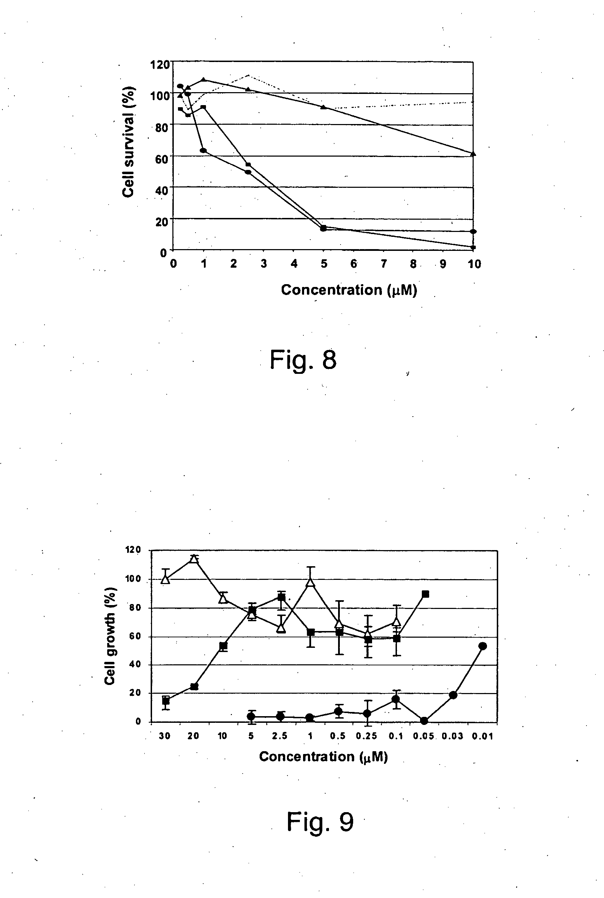

**FIGURE 8****.** NT4(8-13)-Che6 cytotoxicity. HT29 cells were plated in a 96 well microplate and incubated at 37°C in the dark for 4 hours with different concentrations of NT4(8-13)-Che6 (●), scramble NT4(8-13)-Che6 (A) and free Che6 (■). The cells were then washed to remove unbound conjugate and irradiated (except for dark controls) with a total light dose of 3 J/cm². Drug phototoxicity was assessed by measuring cell survival, using the MTT assay 24 hours after irradiation. Negative controls (--) included HT29 cells incubated with the conjugate and not irradiated, cells irradiated and not incubated and untreated cells.
**FIGURE 9****.** NT4(8-13)-MTX cytostatic activity. HT29 cells were plated in a 96 well microplate and treated with different concentrations of NT4(8-13)-MTX (▲), scramble NT4(8-13)-MTX (■) and MTX (●). Inhibition of cell growth was tested by an MTT assay after 72 hours of incubation.

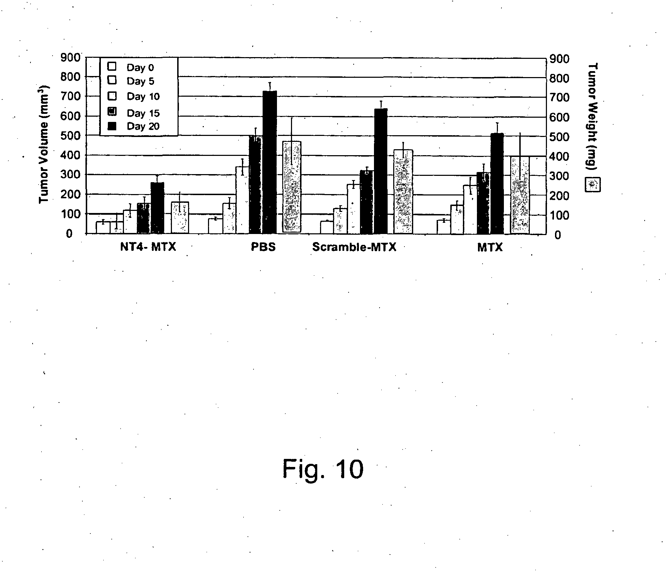

**FIGURE 10****.** *In vivo* tumor growth retardation mediated by NT4(8-13)-MTX. Mice bearing HT29 tumors were randomly divided in four groups and injected i.v. on day 0, 5, 10 and 15 with 500 µg of NT4(8-13)-MTX, 500 µg of scramble-NT4(8-13)-MTX, 40 µg of free MTX and PBS alone. Five days after the last injection, animals were sacrificed and tumors were removed and weighed.

### Examples

### Peptide synthesis

Tetrameric NT(8-13)4-PEG-Biotin [NT4(8-13)-Bio] **1** and tetrameric NT(8-13)4-PEG-Tetramethyl rhodamine [NT4(8-13)-TMR] **2** (Fig. 1) were synthesized using Fmoc strategy with Fmoc-Lys(Biotin)-OH (Novabiochem, Cat. No. 04-12-1237) and Fmoc-Lys(TMR)-OH (Molecular Probes, Cat. No. F11830), respectively, as first coupling step and Fmoc-PEG-OH (Novabiochem, Cat. No. 01-63-0109) as second coupling step. Fmoc-Lys(Fmoc)-OH (Novabiochem, Cat. No. 04-12-1085) was used to build the three-lysine branched core and C-terminal stepwise automated elongation was carried out as follows. A mixture of 5 equiv of N-(9-fluorenyl)methoxycarbonyl (Fmoc) protected amino acid, 5 equiv of 2-(1H-benzotriazole-1-yl)-1,1,3,3-tetramethyluronium hexafluorophosphate (HBTU) (Novabiochem, Cat. No. 01-62-0010) dissolved in DMF, and 10 equiv of N,N-diisopropylethylamine (DIPEA) (Aldrich, Cat.No.387649) were added to the resin beads. The reaction was performed automatically at room temperature for 40 minutes and the coupling step repeated twice. A similar approach was used to synthesize tetrabranched NT(8-13)4-PEG-Chlorine e6 [NT4(8-13)-Che6] **3** and tetrabranched NT(8-13)-PEG-Methotrexate [NT4(8-13)-MTX] **4,** but the side-chain amine group for functional unit coupling was rendered more accessible by stretching with Fmoc-PEG-OH. Thus, NT4(8-13)-Che6 and NT4(8-13)-MTX were synthesized using Fmoc-Lys(Dde)-OH (Novabiochem, Cat. No. 04-12-1121) as first and β-Ala as second amino acid on Novasyn TGR resin (Novabiochem, Cat. No 01-64-0060). The tetramer was then built as above but using Boc-protected instead of the Fmoc-protected Arginine as last amino acid of the neurotensin sequence, so that the last two coupling steps occurred selectively on the side chain arm (Fig. 2).

### Neurotensin Peptide binding to HT29 cell membranes

NT4(8-13), NT4(8-13)-Che6, NT4(8-13)-MTX, NT4(8-13)-Bio and NT4(8-13)-TMR were analyzed for their ability to bind NT receptors (NTR) by measuring inhibition of ¹²⁵I-NT (Amersham Biosciences) binding to HT29 human adenocarcinoma cell membranes, that over-express NTR following the procedure reported in reference 14. A tetrameric peptide carrying scrambled NT aminoacids (LRIPRY), scramble NT4(8-13), was used as control and allowed to verify sequence specificity. All the new molecules maintained biological activity in displacing ¹²⁵I-NT although with different IC50 with respect to the non-conjugated NT4(8-13) (IC50 = 1.9 10⁻¹⁰ M). NT4(8-13)-Bio (IC50 = 1 x 10⁻¹⁰ M) completely maintained the bioactivity of the non-conjugated peptide, whereas peptide IC50 increased in NT4(8-13)-Che6 (IC50 = 2,2 x 10⁻⁶ M), NT4(8-13)-MTX (IC5=5x10⁻⁷ M) and NT4(8-13)-TMR (IC50= 2 x 10⁻⁸ M). On the other hand scramble analogue showed very low affinity (IC50 > 10⁻⁵ M).

These results demonstrate that tetrabranched peptides specific binding ability is maintained after coupling their C-terminus to functional units. However, the size, hydrophilicity and charge of the functional moiety together with the presence of spacer molecules, can modify peptide binding ability.

### Neurotensin Peptide binding and internalization in HT29 cells

Confocal laser microscopy was used to carry out receptor binding assays. For this purpose HT29 cells (human adenocarcinoma cell line, Istituto Zooprofilattico Sperimentale, Brescia, Italy) were incubated for 1 hour at 4°C with NT4(8-13)-TMR (5 µg/ml in PBS - 1% BSA). Similar experiments were performed incubating the same cells with the same concentration of scrambled tetrameric peptide of NT(8-13) coupled with tetramethyl rhodamine or incubating HaCat cells (Human keratinocyte cell line, Istituto Zooprofilattico Sperimentale, Brescia, Italy), which do not express the NTR, with NT4(8-13)-TMR (5 µg/ml in PBS - 1% BSA). After fixing with 4% formalin, the plasma membrane was stained by incubation with concanavalin A-biotin (10 µg/mL in PBS - 1% BSA, Molecular Probes, Cat. No. C21420) at 4°C for 15 min and avidin-FITC (0.5 µg/ml in PBS - 1% BSA, Sigma, Cat. No S3762).

The red fluorescent signal on the plasma membrane (indicated by the arrow in Fig.3) indicates binding of the peptide to membrane receptors (Figure 3A), whereas the absence of red fluorescence in both control images (Figure 3E-F) demonstrated the binding specificity of NT4(8-13)-TMR.

Cell internalization was analyzed by following NT4(8-13)-TMR red signal in HT29 cells over time (Figure 3A-C).

The cells were incubated for 1 h at 4°C with NT4(8-13)-TMR (5 µg/ml in PBS - 1% BSA) to enable peptide binding to membrane receptors, then after washing to remove unbound peptide, the cells were kept at 37°C for different time intervals. At time 0, peptide fluorescence was localized exclusively on the plasma membrane (Fig. 3, panel A, see arrow). Two hours later the peptide was present on the plasma membrane and in the cytoplasm (Figure 3, panel B, arrows), whereas after 4 hours incubation, the peptide was completely internalised (Fig. 3, panel C, arrow). Finally, 18 hours of incubation at 37°C led to complete degradation of the peptide conjugate (Fig. 3, panel D) presumably due to the proteolytic action of lysosomal enzymes.

In order to verify if NT4(8-13)-TMR had a lysosomal localization, HT29 cells were incubated with NT4(8-13)-TMR (5 µg/ml in PBS - 1% BSA) under internalizing conditions (1 h incubation at 4°C with NT4-TMR followed by 4 hours incubation in DMEM at 37°C) and then treated with lysotracker green (Molecular Probes, Cat. No. L7526) to stain lysosomes. In Fig, 4C, colocalization of NT4(8-13)-TMR with lysotracker green was observed, i.e. red signal of the peptide (Fig. 4A) adds to green signal of the lysotracker green (Fig. 4B) to give a yellow signal (arrow).

The ability of the tetrabranched peptide conjugated to a functional unit to be rapidly internalized in cells and still detectable in cells after 4 hours renders the peptide suitable for therapeutic applications of the molecules.

### Detection of NT receptors in human tumors, using NT4(8-13)-Bio

Tumoral and healthy colon and pancreatic tissues were collected from oncological patients undergoing tumor resection. Frozen tissue tek® (Sakura Finetechnical, Tokyo, Japan) embedded tissues were sliced 15 µm thick in a cryostat (Reichert- Jung 2800 frigocut N) and placed on glass slides. Serial sections of the same biopsy were analysed both by hematoxylin/eosin (H&E) light microscopy and by fluorescent confocal microscopy. For immunofluorescent analysis the sections were fixed by immersion in 3% paraformaldehyde in PBS for 15' at room temperature., washed once and stored over night at 4°C with 0.1 M glycine in PBS. After saturation with fetal calf serum, the slices were incubated for 30' at 37°C with NT4(8-13)-Bio (0.5 µg/ml in PBS-BSA 3%) or with the uncorrelated branched peptide (DDHSAV, SEQ ID No. 4)4-Bio (0.5 µg/ml in PBS-BSA 3%), washed twice with PBS and incubated with avidin-FITC (0.5 µg/ml in PBS-BSA 3%) for 15' at room temperature. After washing, the sections were mounted with Mowiol® (Calbiochem, Cat. No. 475904) and sealed with nail polish. Negative controls were carried out incubating the slices (both tumoral and normal tissues) with avidin-FITC without previous incubation with NT4(8-13)-Bio. The fluorescine signal was observed by confocal laser microscopy (BIO-Rad MRC600) with a 488 nm excitation light.

Immunofluorescent analysis with NT4(8-13)-Bio and avidin-FITC performed on frozen sections of biopsy samples revealed a clear preferential binding of the peptide to human colon tumors versus healthy tissue (Figure 5, A-B). Ten colon tumoral biopsies were compared to corresponding normal tissue coming from the same patient. All colon tumoral specimens were positively stained by NT4(8-13)-Bio, whereas only one of the healthy tissues showed a low fluorescent signal. Analogous results were obtained comparing healthy and tumoral pancreatic tissues (Figure 5, C-D). Further experiments were carried out incubating tumoral biopsies with the uncorrelated peptide. The absence of a fluorescent signal (data not shown) observed with the uncorrelated peptide indicated the specificity of the neurotensin multimeric peptide. Finally, a comparative light microscopy analysis of serial sections stained with H and E, revealed that the NT receptor is present mainly in the degenerated mucosa of the tumoral biopsies and only on the cell surface of tumoral cells (data not shown).

### NT receptor regulation

Receptor regulation upon NT4(8-13)-TMR peptide binding was studied by confocal laser microscopy. Receptors were first saturated incubating HT29 cells with a large excess of NT4(8-13)-TMR (100 µg/ml in PBS - 1% BSA) for 1 h at 4°C, in order to saturate receptors and exclude the subsequent binding of NT4(8-13)-Bio detectable with avidin-FITC. Membrane receptors were indeed saturated by NT4(8-13)-TMR, since subsequent incubation with NT4(8-13)-Bio (0.5 µg/ml in PBS - 3% BSA) and avidin FITC (2.5 µg/ml in PBS - 3% BSA) did not produce any detectable green signal (Fig. 7, panel A). When the same cells, treated with NT4(8-13)-TMR, were washed, kept at 37°C in culture medium overnight and newly treated with NT4(8-13)-Bio and avidin-FITC as above, the authors observed that the receptor was again available for ligand binding (Fig. 7, panel B, complete arrow indicate the green signal of NT4(8-13)-Bio-avidin-FITC and the arrow-head the residual red signal of NT4(8-13)-TMR). Regulatory peptide receptors, which are largely G protein coupled receptors, have the important ability to be internalized in cells after ligand binding. This characteristic is one of the features that have made the peptide-bullet strategy a promising approach for tumor therapy^{2,15-19}. Nonetheless, down-regulation of receptors and their depletion from the membrane may dramatically impair the efficiency of a peptide-based therapy.

The ability of NT receptors to be re-exposed on the membrane and still available for peptide binding makes the peptide-based tumor targeting a promising strategy for diagnosis and therapy.

### NT4(8-13)-Che6 cytotoxicity

Photodynamic therapy (PDT) is a promising therapeutic approach to cancer based on selective killing of malignant cells by singlet oxygen, ¹O₂, and other reactive products generated by photoactivated.photosensitizer (PS) molecules, which accumulate in tumor tissue²⁰. Chlorin e6 (Che6) and its derivatives are promising sensitizers for PDT thought their selectivity for cancer cells needs to be improved²¹. Che6 is also known to be cytotoxic for most cells when irradiated at the appropriate wavelength²⁰. Its hydrophobicity enables its non-specific internalization in the cell, causing cell toxicity.

In order to assess the phototoxicity of NT4(8-13)-Che6 conjugate, photokilling experiments were carried out on HT29 cells. HT29 cells (2 x 10⁴/well) were plated in 96-well microplates. After growing for 2 days, the cells were incubated at 37°C in the dark with different concentrations of NT4-Che6 (from 100 µM to 0.1 µM in RPMI with 10% FCS) for 4 hours. The cells were then washed to remove unbound conjugate and irradiated (except dark controls) with a Zeiss KL1500 tungsten halogen lamp (Zeiss, Jena, Germany) equipped with a 600-800 nm band pass filter (Corion, Franklin, MA) and with two optical fibers placed 1 cm from the well. The plate was placed back in the incubator overnight and the phototoxicity of the conjugate was monitored in a MTT assay. Similar experiments were performed with scramble NT4(8-13)-Che6 [(LRIPRY)4-Che6] and with free Che6. Further controls included HT29 cells incubated with the conjugate and not irradiated, cells irradiated and not incubated and untreated cells.

As shown in Fig. 8, Che6 maintained its cytotoxicity after conjugation with tetrameric NT(8-13). The lack of activity of the scrambled analog indicates that the tetrameric NT(8-13) conjugated peptide acquired selectivity and specificity with respect to free Che6. It has been shown that conjugation with branched peptides prevent diffusion of Che6 through cell membranes. In the present invention the authors show that tetrameric NT(8-13) conjugated peptide allows che6 cell internalization through NT receptor-mediated endocytosis, providing che6 cell specific cytotoxicity.

### NT4(8-13)-MTX cytostatic activity

Methotrexate as a folate antagonist is effective in many malignancies since it inhibits dihydrofolate reductase. This causes a lack of reduced folate substrates, therefore impairing synthesis of purine nucleotides, thymidylate, and certain amino acids, which can ultimately lead to cell death. Clinical use of this drug is limited by its dose-related toxic side effects. Moreover, resistance to methotrexate often develops by various mechanisms, including decreased folate carrier-mediated membrane transport²².

To evaluate the cytostatic activity of NT4(8-13)-MTX, in vitro experiments using HT29 cells (Fig. 9) and in vivo experiments using HT29 tumors grafted in nude mice (Fig. 10) were performed.

### In vitro NT4(8-13)-MTXcytostatic experiments

HT29 cells were plated in 0.2 ml aliquots at a density of 5 x 10³ in a 96-well microplate. Different concentrations of NT4-MTX were added 24 hours after plating. Growth inhibition was assessed by MTT assay 6 days later. The same experiments were carried out with the scramble analog of the conjugate and with free MTX.

The same experiments were performed with the scramble analogue [(LRIPRY)4-MTX] and with unconjugated MTX. SW620 (NTR-expressing human colon carcinoma cell line, Istituto Zooprofilattico Sperimentale, Brescia, Italy) and on HCT116 (NTR-expressing human colon carcinoma cell line, Istituto Zooprofilattico Sperimentale, Brescia, Italy cell lines) were also tested with the same procedure.

Fig. 9 shows that conjugated-MTX was only active when the conjugate is an NT tetrameric peptide. Indeed, the scramble analogue conjugated-MTX did not have any cytostatic activity. This result clearly demonstrated that the targeting ability of NT peptide is necessary to achieve the therapeutic efficacy of conjugated-MTX. Hence the same may be true for the acquired specificity of MTX toxicity, once linked to a specific peptide carrier, as for Che6 cytotoxicity. Conjugation of these toxic moieties with branched peptide molecules makes them pro-drug-like compounds, which can no longer be transported across plasma membranes by the mechanisms of the corresponding free drugs and can only be 'activated' via peptide-receptor binding that mediates their specific translocation inside the cell. This feature profoundly decreases the non-specific toxicity of the drug. Moreover, receptor-mediated transport of the peptide across the target cell membrane, may help to bypass drug-resistance mechanisms based on specific membrane transporters, like that of MTX. The same results were obtained on SW620 and on HCT116 cell lines (not shown).

### In vivo experiments

Female CD-1 nude mice (Charles River Laboratories Inc., Wilmington, MA) 6-7 weeks of age (mean weight of 25 g), were injected subcutaneously in the right flank with of 2 x 10⁶ HT29 cells. When tumors reached a diameter of 4-5 mm, the mice were randomly divided into groups (at least 4 mice/group) and repeatedly injected iv (on days 0, 5, 10 and 15) with 500 µl of the following solutions:
group I: 1 mg/ml NT4-MTX (4.05 µmol/kg) in PBS;
group II: 1 mg/ml scramble (LRIPRY)4-MTX (4.05 µmol/kg) in PBS, control;
group III: 72 µg/ml free MTX (4.05 µmol/kg), control;
group IV: PBS, control.

Tumor volumes were measured daily with a caliper using the following formula: Volume = length x width² x Π/6. The tumor volumes for each group were pooled to plot mean tumor growth curves. At the end of the experiment, the mice were sacrificed. Tumors were removed and weighed and the weights compared between treated and control mice. NT4(8-13)-MTX prevented tumor growth when compared to all control mice (Fig. 10). The reduction was evident soon after the first injection and the volume of tumors from animals treated with NT4(8-13)-MTX remained about one third that of tumors from mice injected with PBS, over the whole treatment. A slight decrease in tumor growth was found in animals treated with MTX alone as well as in mice treated with scramble NT4(8-13)-MTX. Tumor weight, measured at the end of the experiment, was almost the same in the three control groups (PBS, scramble NT4(8-13)-MTX, MTX alone) and significantly reduced in the NT4(8-13)-MTX-treated group (Fig. 10). These results demonstrate that the specific action of the NT4(8-13)-MTX molecule as shown *in vitro* is confirmed *in vivo.*

In addition, NT4(8-13)-MTX-treated animals lost less than 10% of body weight and no mice death was observed in such group, excluding toxicity of NT4(8-13)-MTX at the dose administered.

It should be noted that NT4(8-13)-MTX, scramble NT4(8-13)-MTX and MTX were injected at the same molar concentration. The administered concentration represents a MTX dose that was about one tenth of the dose commonly used for the treatment of tumor xenografts in nude mice²³, suggesting an increased potency of the NT4(8-13)-MTX molecule.

### Bibliography

1. Reubi JC. J Nucl Med. 1995; 36(10): 1825-35.
2. Reubi JC. Endocr Rev. 2003; 24(4): 389-427.
3. Langer M and Beck-Sickinger AG. Curr Med Chem Anti-Canc Agents. 2001; 1(1): 71-93.
4. Garcia-Garayoa E et al., Nucl Med Biol. 2001; 28(1): 75-84.
5. Hillairet De Boisferon M et al., Bioconjug Chem. 2002; 13(3): 654-62.
6. Achilefu S et al., JMed Chem. 2003; 46(15): 3403-11.
7. Bergmann R et al., Nucl Med Biol. 2002; 29(1): 61-72.
8. de Visser M et al., Eur JNucl Med Mol Imaging. 2003; 30(8): 1134-9.
9. Buchegger F et al., JNucl Med. 2003; 44(10): 1649-54.
10. Morinville A et al., IntJBiochem Cell Biol. 2004; 36(11): 2153-68.
11. Reubi JC, Mäcke HR, Krenning EP. JNucl Med. 2005; 46: 67S-75S
12. Reubi JC, Waser B, Friess H, Buchler M, Laissue J. Gut. 1998; 42(4):546- 550
13. Nagy AN, Schally AV. Biol Rep 2005; 73: 851-859
14. Bracci L et al., J Biol Chem. 2003; 278(47): 46590-5.
15. Nouel D et al., J Neurosci. 1997; 17(5): 1795-803.
16. Mantyh PW et al., Science. 1995; 268: 1629-32.
17. Roettger BF et al., J Cell Biol. 1995; 128: 1029-41.
18. Mazella J et al., Brain Res. 1991; 564(2): 249-55.
19. Ottaway CA. Regul Pept. 1992; 41(1): 49-59.
20. Yarmush ML et al., Crit Rev Ther Drug Carrier Syst. 1993; 10(3): 197-252.
21. Hamblin MR et al., Cancer Res. 2001; 61(19): 7155-62.
22. Zhao R, Goldman ID. Oncogene. 2003; 22(47): 7431-57.
23. Wosikowski K et al., Clin Cancer Res. 2003; 9(5): 1917-26.

## Claims

1. A multimeric peptide having the general formula A or B: 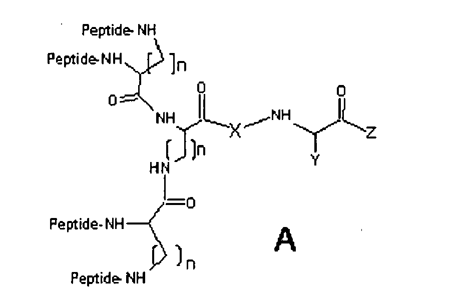 or 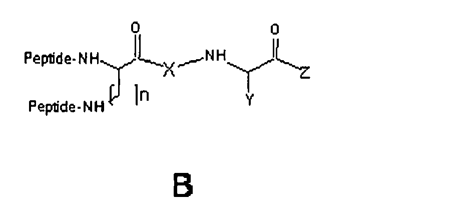 wherein
n = 1 to 5,
x= 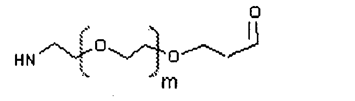 wherein m = 1 to 27,
or 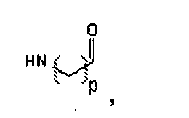 wherein p= 1 to 5,
Y = 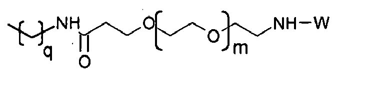 wherein m = 1 to 27 and q= 1 to 5
or 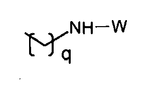 wherein q= 1 to 5,
or 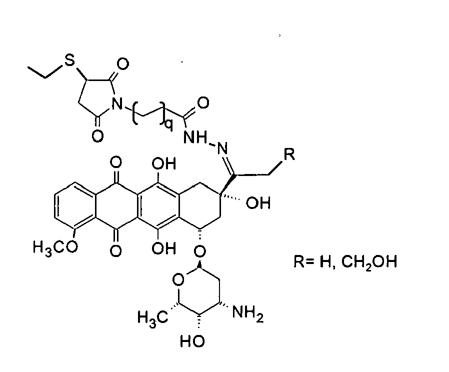 wherein q= 1 to 5
W = 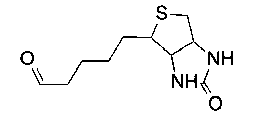 or 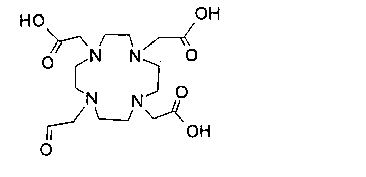 or 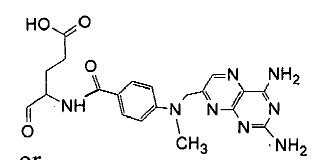 or 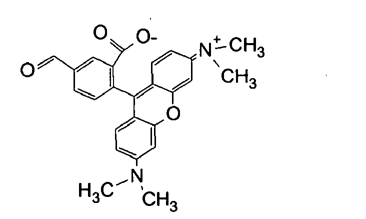 or 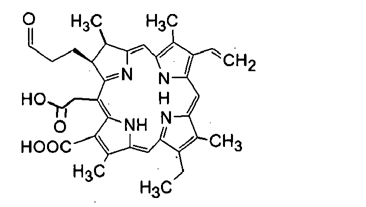 and Z = NH₂ or OH.
wherein each peptide has a Neurotensin (NT) derived sequence, for use as an anti-tumoral agent.

2. The multimeric peptide according to claim 1 for use as an anti-tumoral agent, wherein each peptide having a Neurotensin (NT) derived sequence has the following amino acid sequence: QLYENKPRRPYIL or RRPYIL.

3. The multimeric peptide according to claim 1 or 2 for use as an anti-tumoral agent being conjugated with a functional unit.

4. Pharmaceutical composition comprising the multimeric peptide according to claim 1 to 3, or a pharmaceutically acceptable and efficient salt thereof and diluents, and/or solvents and/or carriers and/or eccipients.

5. Use of the multimeric peptide according to claims 1 to 3 for tumor diagnostics.

6. Method to in vitro diagnose a tumor in a sample comprising the steps of:
a) incubating the sample with the multimeric peptide according to any of claims 1 to 3 in proper conditions to allow the specific binding of the peptide to the sample, and
b) detecting the bound peptide.

## Patentansprüche

1. Multimeres Peptid der allgemeinen Formel A oder B: 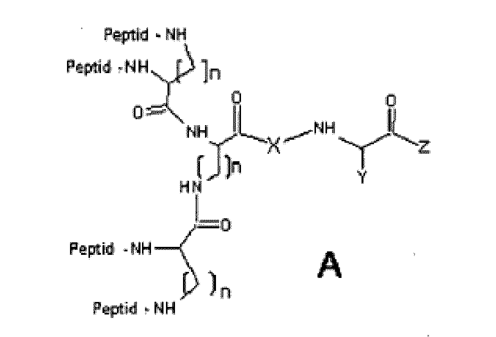 oder 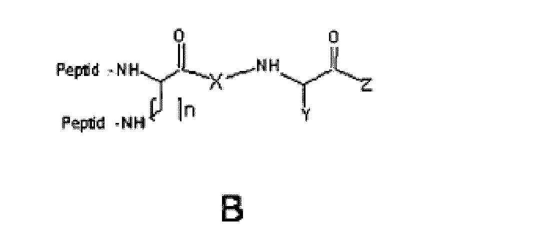 wobei n = 1 bis 5,
**X =** 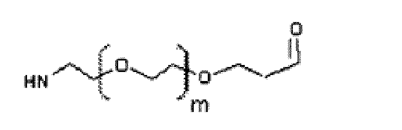 wobei m = 1 bis 27,
oder 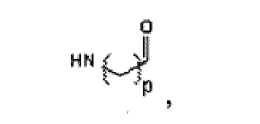 wobei p = 1 bis 5,
**Y =** 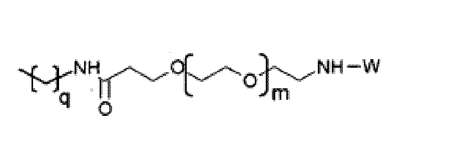 wobei m = 1 bis 27 und q = 1 bis 5,
oder 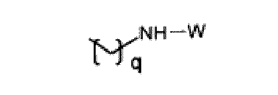 wobei q = 1 bis 5,
oder 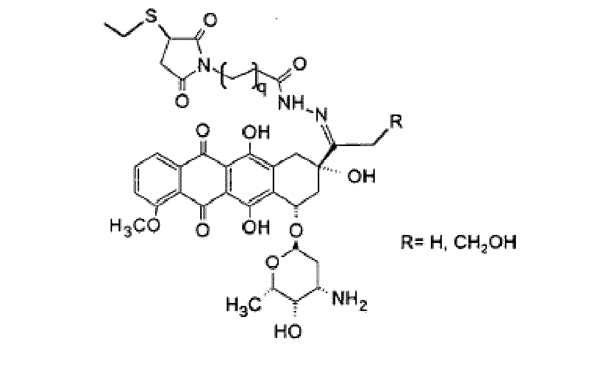 wobei q = 1 bis 5, 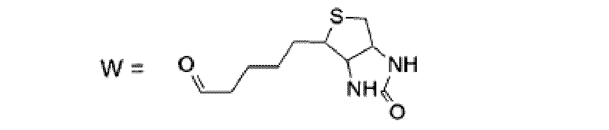 oder 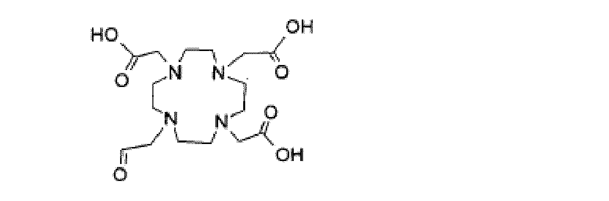 oder 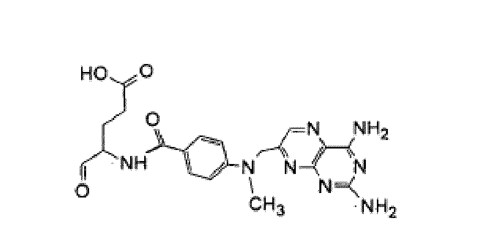 oder 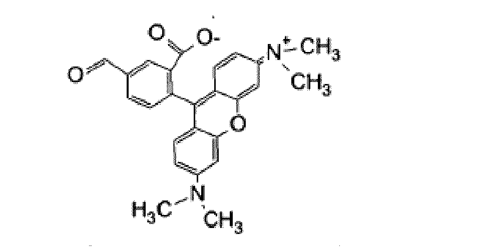 oder 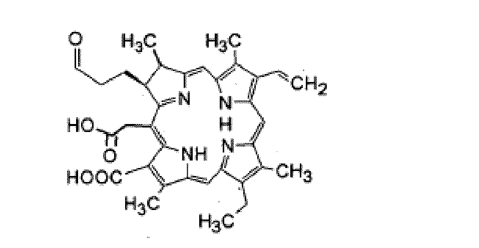 und Z = NH₂ oder OH,
wobei jedes Peptid eine vom Neurotensin (NT) abgeleitete Sequenz aufweist, zur Verwendung als ein Antitumormittel.

2. Multimeres Peptid nach Anspruch 1 zur Verwendung als ein Antitumormittel, wobei jedes Peptid, das eine vom Neurotensin (NT) abgeleitete Sequenz aufweist, die folgende Aminosäuresequenz aufweist: QLYENKPRRPYIL oder RRPYIL.

3. Multimeres Peptid nach Anspruch 1 oder 2 zur Verwendung als ein Antitumormittel, welches mit einer funktionellen Einheit konjugiert ist.

4. Pharmazeutische Zusammensetzung, umfassend das multimere Peptid nach Anspruch 1 bis 3 oder ein pharmazeutisch unbedenkliches und wirksames Salz davon und Verdünnungsmittel und/oder Lösungsmittel und/oder Trägerstoffe und/oder Hilfsstoffe.

5. Verwendung des multimeren Peptids nach Anspruch 1 bis 3 zur Tumordiagnostik.

6. Verfahren zur In-Vitro-Diagnose eines Tumors in einer Probe, umfassend die Schritte:
a) Inkubieren der Probe mit dem multimeren Peptid nach einem der Ansprüche 1 bis 3 in passenden Bedingungen, um die spezifische Bindung des Peptids an die Probe zu ermöglichen; und
b) Erfassen des gebundenen Peptids.

## Revendications

1. Peptide multimère ayant la formule générale A ou B : 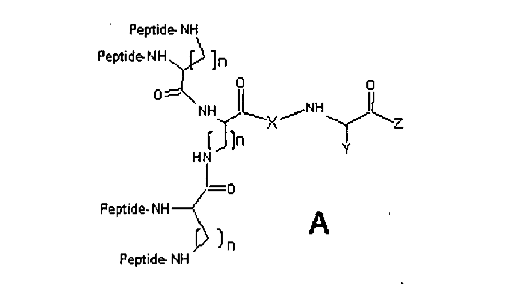 ou 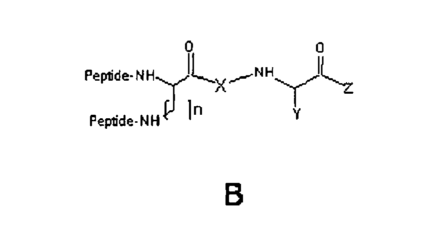 dans laquelle
n = 1 à 5,
X = 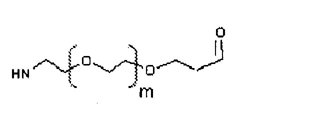 dans laquelle m = 1 à 27,
ou 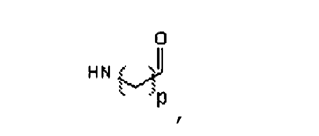 dans laquelle p = 1 à 5,
Y = 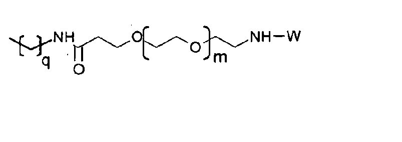 dans laquelle m= 1 à 27 et q = 1 à 5
ou 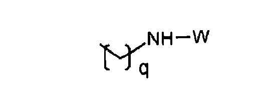 dans laquelle q = 1 à 5,
ou 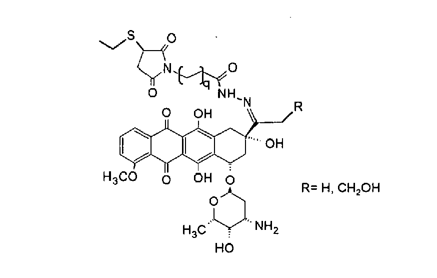 dans laquelle q = 1 à 5
W = 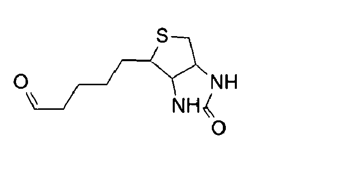 ou 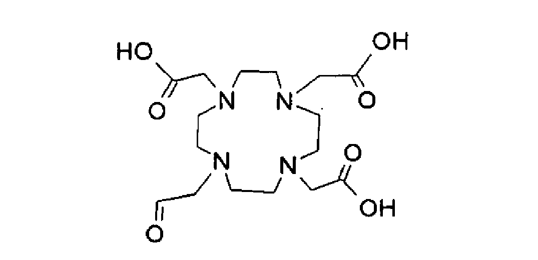 ou 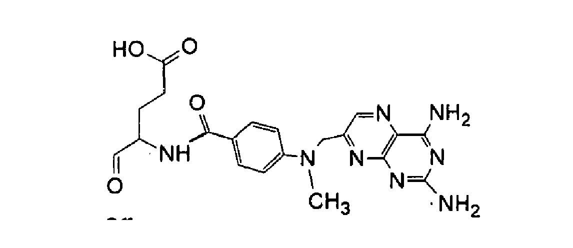 ou 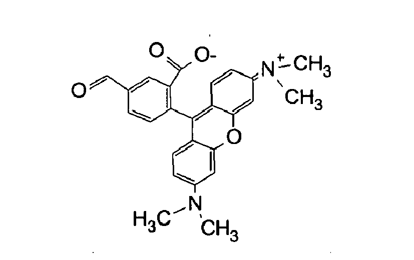 ou 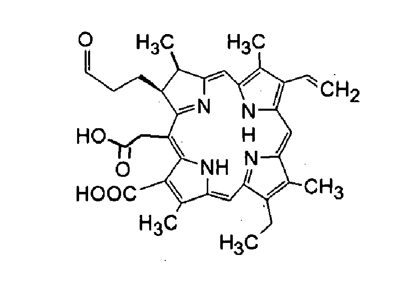 et Z = NH₂ ou OH
dans laquelle chaque peptide a une séquence dérivée de la Neurotensine (NT), à utiliser comme agent antitumoral.

2. Peptide multimère selon la revendication 1 à utiliser comme agent antitumoral, dans lequel chaque peptide ayant une séquence dérivée de la Neurotensine (NT) a la séquence d'acides aminés suivante : QLYENKPRRPYIL ou RRPYIL.

3. Peptide multimère selon la revendication 1 ou 2 à utiliser comme agent antitumoral étant conjugué avec une unité fonctionnelle.

4. Composition pharmaceutique comprenant le peptide multimère selon la revendication 1 à 3, ou un sel pharmaceutiquement acceptable et efficace de celui-ci et des diluants, et/ou des solvants, et/ou des supports et/ou des excipients.

5. Utilisation du peptide multimère selon les revendications 1 à 3 pour des diagnostics de tumeur.

6. Procédé pour diagnostiquer in vitro une tumeur dans un échantillon comprenant les étapes consistant à :
a) incuber l'échantillon avec le peptide multimère selon l'une quelconque des revendications 1 à 3 dans des conditions appropriées pour permettre la liaison spécifique du peptide à l'échantillon, et
b) détecter le peptide lié.
